# EUROPEAN PATENT APPLICATION

(11) **EP 2 098 590 A1**
(43) Date of publication of application: **09.09.2009**
(21) Application number: 08102277.4
(22) Date of filing: 04.03.2008
(51) Int. Cl.: C12N 5/10

(54) **Cell line from Rousettus as host cell for pathogen amplification**

(71) Applicant: ProBioGen AG, 13086 Berlin (DE)
(72) Inventor: Jordan, Ingo, 13156, Berlin (DE); Horn, Deborah, 13051, Berlin (DE); Sandig, Volker, 13158, Berlin (DE)
(74) Representative: Zwicker, Jörk

(57) **Abstract**

The present invention relates to immortalized cell lines from fruit bats suitable for amplification of pathogens, preferably viruses, itsproduction and use for diagnostic or therapeutic purposes.

## Description

The present invention relates to immortalized cell lines from fruit bats suitable for amplification of pathogens, preferably viruses, itsproduction and use for diagnostic or therapeutic purposes.

### Background of the Invention

Within the mammalia bats are second only to rodents in species diversity. The taxonomic order of bats is Chiroptera, further classified into the suborders Megachiroptera and Microchiroptera, usually referred to as fruit bats or megabats and insectivorous bats or microbats, respectively. The Megachiroptera contains a single family, the Pteropodidae, whereas the Microchiroptera are subdivided into seven superfamilies comprising a total of seventeen families.

Greater taxonomic complexity of microbats is mirrored in their worldwide geographic distribution that includes temperate climate zones and the Americas compared to megabats that are confined to tropical and subtropical regions of the Eastern Hemissphere.

Microbats have tails and one clawed finger on each wing, megabats generally have no tail and two clawed fingers on each wing. Megabats have simple ears with the rim of the pinnae forming a closed ring and simple snouts without the complicated nose leaves supporting production of echolocation signals. Microbats are insectivorous, hunt small animals or feed on blood. Megabats are frugivorous or nectarivorous.

Microbats but not megabats are able to perform echolocation for orientation and to avoid obstacles. Megabats rely on their acute sense of vision; indeed, only in megabats (but not in microbats) neuronal organisation connecting retina and mid-brain appears just as advanced as it is in primates (Pettigrew 1986 in Science 231, 1304-1306). As the single exception within the megabats Rousettus aegyptiacus is also capable of sonar orientation. However, the echolocation system of Rousettus is not related to the sophisticated laryngeal echolocation of the microbats (Springer 2001 et al. in Proc. Natl. Acad. Sci. U.S.A. 96, 6241-6246; Holland et al. 2004 in J. Exp. Biol. 207, 4361-4369). It is the result of convergent evolution and comparatively simple where the emitted signal is produced as low-energy clicks by the tongue. Furthermore, Rousettus ears lack the muscles and innervation required for self-deafening to improve information content of the reflected sound.

The profound differences between microbats and megabats in geographical distribution, behaviour, anatomy and physiology stimulated a controversial discussion whether the flying mammals truly are monophyletic. Tree building based on mitochondrial (Lin and Penny 2001 in Mol. Biol. Evol. 18, 684-688), genomic sequences incorporating the scarce available fossil data (Springer et al. 2001; Teeling et al. 2005 in Science 307, 580-584) and supertree algorithm (Emonds et al. 2007 in Nature 446, 507-512) suggests that bats have evolved from a common ancestor with megabats in a distinct clade. Fossilation of the most primitive bat found to date (a bat already capable of flight but not yet of echolocation, still with claws on all digits) is dated to have occured 52.5 million years ago (Simmons et al. 2008 in Nature 451, 818-822). Powerful larnygal echolocation has evolved subsequently only once but was lost in the megabats where it was reinvented in its simple form only by Rousettus.

Bats are vectors and reservoir for a number of important and emerging viruses, including members of the filoviridae (such as Marburg and Ebola virus), paramyxoviridae (such as Nipah virus), rhabdoviridae (such as rabies and European bat lyssavirus) and coronaviridae (the SARS-CoV).

Most surprising is the fact that bats appear not or only minimally to be affected by a variety of pathogens that usually are fatal to vertebrates.

For example, Ebola virus was detected in wild megabats collected at sites near to infected gorilla and chimpanzee carcasses. The megabats were positive for genomic RNA sequences from or antibodies against Ebola but did not display any disease symptoms (Leroy et al. 2005 in Nature 438, 575-576). Transfer of virus probably occurs via fruit contaminated by the megabats during foraging.

Nipah and Hendra viruses are associated with high mortality but, again, in megabats that serve as reservoirs there are no symptoms (Reynes et al. 2005 in Emerg. Inf. Diseases 11, 1042-1047). Microbats appear not to carry Nipah or Hendrah viruses.

According to the World Health Organisation, 55000 human deaths from rabies are reported annually. Rabies is invariably fatal to mammals with extremely rare and unusual exceptions: in spotted hyenas of the Serengeti a special, possibly attenuated strain of rabies has established endemic persistence (East et al. 2001 in Proc. Natl. Acad. Sci. U.S.A. 98, 15026-15031), and a single wild oncilla with antibody titers suggestive of exposure to rabies virus but otherwise clinically inapparent was captured in Bolivia (Deem et al. 2004 in J. Wildlife Diseases 40, 811-815). Certain bat species, however, frequently are found to carry rabies virus without overt symptoms (for example Poel et al. 2005 in Emerging Inf. Dis. 11, 1854-1859 for European bats and Messenger et al. 2002 in Clinical Inf. Dis. 35, 738-747). Rabies ecology is complicated and many wild and domestic animals are vectors depending on country and geographic region. In Latin America main vectors and reservoirs for rabies appear to be hemovorous (vampire) bats and dogs (Ito et al. 2001 in Virology 284, 214-222). Insectivorous bats are important vectors for cryptic rabies in developed countries: transmission of virus after encounter with a bat has not been realized until it is too late for post-exposure prophylaxis (Feder et al. 1997 in Lancet 350, 1300). Most fascinating and further highlighting significance of bats for rabies dissemination is a phylogenetic analysis suggesting that possibly an insect rhabdovirus transferred into an insectivorous bat, evolved into bat lyssaviruses and from there repeated further host switching into the carnivora has allowed rhabies virus to emerge as it is known in contemporary mammals (Badrane and Tordo 2001 in J. Virol. 75, 8096-8104).

Spread of an agent causing severe acute respiratory syndrome (SARS) in the human population nearly created a pandemic in 2002/2003. With rapid identification of the pathogen, a coronavirus named SARS-CoV, it was subsequently realized that SARS-CoV entered the human populatian via a zoonotic event at a Chinese meat market with civet cats as source (Guan et al. 2003 in Science 302, 276-278). Recent analysis indicate that fruit bats and microbats are reservoir for SARS-CoV (Li et al. 2005 in Science 310, 676-679); again, infection may have initiated via spillover to other species after a pathogen evolved in bats where it does not cause disease.

The fatal suitability of bats as vectors may be due to a coincidental combination of behavioural, evolutionary and physiological properties:

Many bats tend to live in large communities at high population density thus facilitating repeated exposure, spread and maintenance of certain pathogens. Spread within a roost may be further enhanced or modulated if the act of echolocation generates an aerosol of pathogens suspended in saliva and mucus from mouth or nose of the animals. Transmission with aerolized pathogens may cause infection with low viral loads or unusual entry into the recipient (for example, mucosal infection with rabies or flavivirus rather than parenteral via bite or insect vector) and this may lead to persistent and subclinical infection.

Bats can fly and thus a carrier of a disease may cover large areas or gain more easily access to human shelters to transmit a pathogen. This propability for transmission is further increased by the long lifespan of bats.

Self-powered flight places a large burden on efficient metabolism. To conserve energy, some bats hybernate or reduce body temperature even for daily sleep. The adaptation to high metabolic rates, hypothermia by itself and possibly intermittent depression of the innate and adaptive immune system due to these adaptations may help viruses to establish subclinical persistence.

Finally, bats are an evolutionary very old order of the mammalia class. Difficult and enigmatic to trace via fossil and molecular records, the latest common origin of the various bat species has been estimated to have lived 89 million years ago in the late Cretaceous period (Bininda-Emonds et al. 2007 in Nature 446, 507-512). Major speciation of bats appears to have started within the K-T boundary (Teeling et al. 1995 in Science 307, 580-584), a geological signature assumed to have been caused by a catastrophic event approximately 65 million years ago. The K-T boundary separates sediments from the Cretatious and Tertiary periods and coincides with the extinction of dinosaurs and increase of plant and insect diversity -- liberating ecological niches and providing new sources for foraging. Being an evolutionary old clade may translate into two properties with respect to suitability as disease vectors: their adaptive immune system may react to certain pathogens very differently compared to the immune system of most mammalia, and some of the more dangerous zoonotic agents may have decreased pathogenicity towards bats due to co-evolution with the flying mammals as reservoir.

In summary, bats are fascinating animals with significant impact on the infectious disease ecology, both as reservoir and vector. Shadowed by the huge taxonomic diversity of microbats, megabats form a unique clade within these unique mammals. Specifically megabats have been implicated as disease carriers for important pathogens such as filoviridae.

Many macroscopic determinants for suitability of bats as vectors have been discussed above. The individual cell also has been shaped by these circumstances. For example, energy expenditure of self-powered flight is high and physiology and biochemistry has to adapt to the increased metabolic requirement. It has recently been suggested that this adaptation extends to the individual cell (Organ et al. 2007 in Nature 446, 180-184): the genome of birds and bats is surprisingly small compared to other vertebrates. A small genome translates into a small nucleus and thus into a small cell volume. Diffusion of dissolved gases, nutrients and metabolites is more efficient in small cells.

On the other side of the evolutionary equation, some viruses that are serious threats today may have adapted to bats as reservoirs and thus evolved to find suitable receptors, cellular cofactors for genome replication, viral protein processing and maturation, and virion morphogenesis and egress.

Thus, properties of cell lines derived from bats may be of profound use from an industrial perspective: it is assumed that important infectious agents find a unique environment in bat cells especially from the Megachiroptera with respect to host range, productivity and formation or avoidance of cytopathic effect. An immortalized cell line derived from a Megachiroptera would be extremely beneficial to virus and vaccine research and to production of prophylactic or therapeutic vaccines or viral vectors. Furthermore, an immortalized cell line derived from a Megachiroptera may allow cell based assays for isolation of pathogens yet insufficiently characterized for PCR or serological diagnosis. Furthermore, we propose using bat cells with their naturally high metabolic advantages as industrial producer cells. To utilize and explore these properties one first has to generate such a cell line. With such a cell line, preferrably aided by modern proteomics and genomics, it is identify nodes and factors in the biochemical pathways that are instrumental for transfer of bat cell properties to common producer cells for viruses or proteins such as Vero or CHO. It is also possible to identify and characterize properties evolution has shaped in bats with respect to virus susceptibility and degree of or resistance against an infection. These factors, and the properties they confer, are transferred to suitable host cells from other species, avian, insect or human for example, for generation of therapeutic molecules, attenuated or targeted viruses, and viral vectors in therapeutic or prophylactic approaches.

### Summary of the Invention

Thus, an immortalized cell line from the Megachirpteran Rousettus aegyptiacus has been provided. The cell lines have been generated by liposomal transfection of E1 genes from human adenovirus serotype 5 but any means of immortalization via cell cycle induction and inhibition of apoptosis, either by direct targeting of the pathways or random events is possible. A representative cell line, AGE1.R06E has been deposited with the DSMZ under the accession number ACC...

We also provide data that indicate surprisingly effecient permissivity and replication potential for the highly attenuated modified vaccinia Ankara (MVA) virus.

The invention thus provides
(1) a cell line derived from a bat (Chiroptera) cell immortalized by a defined mechanism (i.e., an immortalized bat cell line);
(2) a preferred embodiment of the cell line of (1) above, which is a primary cell of Megachiroptera Rousettus aegyptiacus immortalized by an adenoviral E1 gene, preferably carrying nt ... to ... of SEQ ID NO:1, most preferably is cell line AGE1.R06E deposited under DSM ACC...;
(3) a method for preparing the cell line as defined in (1) or (2) above, which comprises immortalizing a starting bat cell;
(4) a method for producing a microbial agent on a cell line as defined in (1) or (2) above, which comprises contacting said cell line with
   (i) the microbial agent, including infection with the microbial agent, cultivating the infected cells, harvesting the cells or the culture supernatant to retrieve the microbial agent; or
   (ii) with a nucleic acid sequence encoding said microbial agent, including transfection or transduction with an expression plasmid or *in vitro* transcribed RNA, cultivating the transfected cells, harvesting the cells or the culture supernatant to retrieve the microbial agent;
(5) a method for diagnosis, identification, retrieval or rescue of a microbial agent which includes exposing the cells from a cell line as defined in (1) or (2) above to medium or biological samples suspected to contain a certain microbial agent; and
(6) the use of a cell line as defined in (1) or (2) above for production, diagnosis, identification, retrieval or rescue of a microbial agent, or for identification of at least one factor important for replication of or resistance against a microbial agent, and application or exogenous expression of this factor or these factors in a cell that is not isolated from a chiropteran.

### Short Description of the Figures

Fig. 1: Isolation of cells from a fetus of Rousettus aegyptiacus.

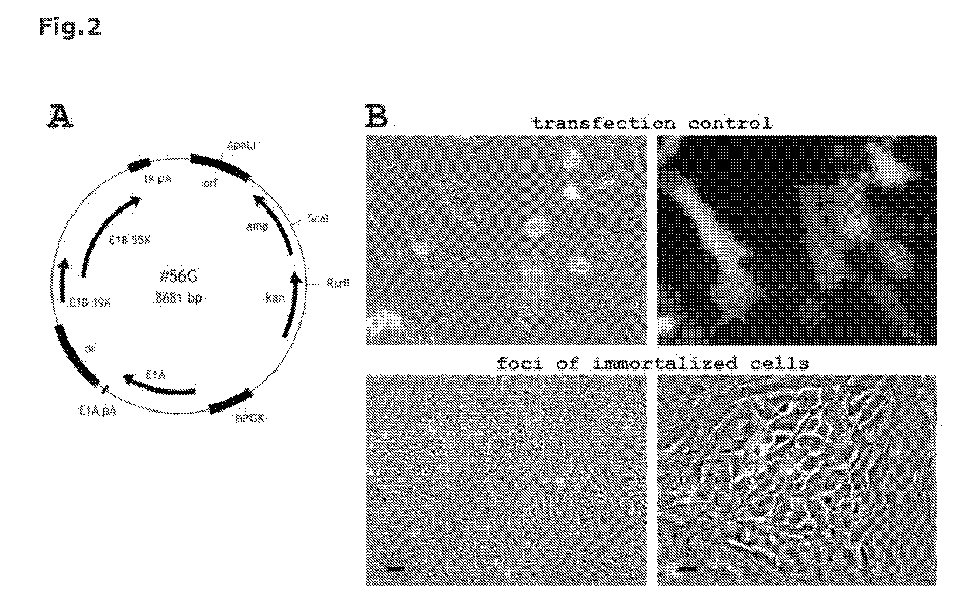

Fig. 2: (A) Plasmid used for immortalization of primary Rousettus cells. Shown are functional elements (E1A and E1B genes), location of expression cassettes (driven by human EF promoter for E1A and thymidine kinase promoter of herpes simplex virus for E1B). Also shown are target sites for restricion enzymes used to linearize plasmid prior to transfection. The plasmid is not equipped to express any selection markers in eukaryotic cells. (B) GFP positive control in upper panel to demonstrate successfull transfection of primary cells. Foci of immortalized cells in lower panel still embedded in multitude of primary cells.
Fig. 3: Appearance of cell lines after immortalization surprsingly resembles morphology and growth properties of the source material. Also shown is a senescent cell that does not proliferate and is lost with passaging of the established Rousettus cell lines. Note highly unusual neuronal cell line in bottom panel.
Fig. 4: Early passage history of three of the established cell lines. Note differences in cell proliferation rates that increase for R06E at week 30. The neuronal cell line R05R does not exhibit strong proliferation.
Fig. 5: Immunofluorescence of Rousettus cell lines R05T and R06E demonstrating successfull stable integration of expression plasmid for E1 genes. CR also expresses adenovirus E1A genes and serves as positive control, BHK as negative control.
Fig. 6: Appearance of cytopathic effect after infection with modified vaccinia Ankara at a multiplicity of infection of 0.1. Note heavy damage to cell layers in highly susceptible avian (CR and CS) and Rousettus (R05T and R06E) cell lines.
Fig. 7: Yields for MVA on various cell lines.
Fig. 8: The Rousettus cell line R06E is susceptible to induction of antiviral pathways by poly(I:C) chemical inductor. CS also is susceptible and serves as positive control, Vero is known to be refractory and serves as negative control.

### Detailed Description of the Invention

"Immortalized", "immortalized cells" and "immortalized cell line" according to the present invention relates to a cell or cell line which has been transfected/transformed by certain functional DNA sequences conferring the potential for at least 200 passages, preferably unlimited number of passages, i.e. immortality, to the respective starting cells.

Cells intended for pharmaceutical purposes such as vaccine or vector production should fulfill a set of strict requirements to minimize risk to recipients (Guidance for Industry, FDA/CBER in September 2006). Most important is compatibility with the Defined Risk Approach (DRA), an algorithm that attempts to estimate risk to recipients of therapeutic proteins or vaccines from certain facts about a cell line such as nature of source material, mechanism of immortalization, tumorigenicity, and characterization for adventitious agents and endogenous retroviruses. It is highly desirable to generate a bat cell line that is compatible with these requirements. The cell line of aspect (i) of the invention fulfills said requirement.

The first steps towards an immortal cell line are extremely important. A cell line should not be derived from a natural tumor or by repeated passaging until a spontaneous transformation event has occured (or been induced with chemical carcinogens) unless the lesion within the biochemical network is fully characterized and exhaustively determined. The reason is that often vaccines are only minimally purifified to maintain viability of the therapeutic component and thus may become contaminated with genomic sequences or protein or mobilized elements from the host cell. The molecular transformation mechanism in spontaneous or natural tumors often cannot be completely elucdiated. The risk from transfer of an unknown mutated oncogene or oncovirus cannot be estimated and justified especially in the light that vaccines often are given a healthy population at a very young age.

For research purposes it is also desirable to know what biochemical pathways have been affected in generation of a cell line.

Immortalization by designed and focused manipulation of biochemical pathways, either by knowledgeable insertion or activation of factors or deletion of cellular mechanism, is compatible with defined risk approaches. To minimize risk, the manipulation should preferably affect or introduce factors of low aggressivity, for example oncogenes that are not pleotropic to an extent that immortalization, transformation, self-sufficiency with respect to growth factors, and metastasis are mediated by a single event. The factor or manipulation should preferably avoid to induce mutagenesis in the host genome.

For example, SV40 polyomavirus large T antigen is a multifunctional protein which affects both checkpoint control in G1 of the cell cycle and p53 activity. Therefore, large T readily immortalizes and transforms multiple mammalian tissues of rodent and human origin. Therefore, this approach is considered aggressive.

Often induction of cell cycle progression (a required event for generation of a stable cell line) induces apoptosis as innate defense mechanism of multicellular organism. We have decided to introduce two new genes that affect checkpoint control of the cell cycle and induction of apoptosis via separate factors: a required simultaneous transfer event of two distinct factors for transformation dramatically decreases any theoretical risk for the vaccinee. An identical desired effect may also be obtained by deletion of cellular genes, for example by knock-out or insertional mutagenesis of the apoptosis mediator and concomitant introduction of the factor affecting cell cycle control.

We have chosen to immortalize bat cells with the E1-gene from human adenovirus serotype 5. Adenoviruses (AdV) are well characterized, naked (non-enveloped) ubiquitous viruses. For the most common serotypes Ad2 and Ad5 the seroprevalence in the human population approaches 90%. Replication incompetent versions of these viruses are used as gene therapy and vaccine vectors in trials with human patients. Genes from the E1 region of human Adenovirus 5 have been used to transform some specific human cells in vitro (293 and PER.C6 cell lines; Fallaux, F.J. et al., Hum. Gene Ther. 9:1909-17 (1998); Graham, F.L. et al., J. Gen. Virol. 36:59-74 (1977)). Mammalian E1-transformed cell lines have been used for the production of live purified adenovirus vectors in clinical trials. With careful monitoring of the amount of contaminating cellular DNA in a vaccine preparation and its size, the transforming genes of Ad5 are not considered a safety hurdle (Vaccines and Related Biological Products advisory committee, session from May 16, 2001).

The general process is inefficient compared to stronger multifunctional oncogenes such as SV40 large T antigen. Based on the observation that HEK 293 cells show neuron specific markers and PER.C6 are of neuroectodermal origin it was suggested that Ad5 E1-based transformation is limited to neuronal cells (Shaw et al. Faseb J 16(8): 869-71(2002)). We have observed in earlier experiments that immortalization of human cells with E1-genes induces a shift in gene expression towards epithelial patterns (Sandig and Jordan 2006 in Drug Testing In Vitro, ed. Marx and Sandig, WILEY-VCH, Weinheim, chapter 7). Obtaining three cell lines of different morphologies (fibroblast, endo/epithelial and neuronal) is surprising and may reflect a property of chiropteran cells. Especially rescue of a cell line that in morphology and growth kinetics indicates that a neuronal cell line has been generated is an achievement considering the well known difficulties in maintaining primary neuronal cultures (for example, see Brewer and Cotman 1989, Brain Res. 494, 65-74, who employ hypoxic culture techniques in presence of anti-oxidants in extremely small culture volumes).

Adenoviruses replicate in the nucleus of the infected cell. Because quiescent host cells are not permissive for a full viral life cycle adenoviruses have evolved mechanism to force cells into S-phase. To maximize burst size of progeny viruses they have also evolved mechanism to evade apoptosis as a response of the host cell to capsid penetration and viral replication. The genomic region that mediates both cell cycle progression and inhibition of apoptosis is the E1 region.

The E1 region actually consists of two distinct expression cassettes, E1A and E1B, arranged in tandem and each equipped with its own promoter and polyadenylation site. At least three proteins are translated from the E1A primary transcript by alternative splicing. Among others, E1A proteins have been found to disrupt RB/E2F complexes and to interfere with the p300 and CBP transcriptional co-activators. The escape of E2Fs from the RB repressor induces progression of the cell cycle from G1 to S phase, whereas the E1A/p300 complex induces apoptosis via several pathways (Putzer, B.M. et al., Cell Death Differ. 7:177-88 (2000)), including repression of transcription of MdM2, a negative regulator of the key sensor for apoptosis, p53.

As E1A sensitizes cells to TNF-induced apoptosis it is considered an antitumor agent, and it is used in experimental approaches for tumor treatment (Lee, W.P. et al., Cancer Res. 63:6229-36 (2003)).

Furthermore, acting as a transcription modulator it drives cells towards de-differentiation, a feature advantageous to a potential cell substrate.

The E1B region encodes two open reading frames on a bicistronic mRNA, the 21K and 55K proteins. The 55K protein binds to p53 and thus turns the pro-apoptotic transcriptional activator into a repressor. The 21K protein complements this anti-apoptotic activity by binding to Bax, thus maintaining integrity of the mitochondrial membrane and preventing the release of cytochrome C. This protein is essential to drive adherent cells towards substrate independent growth and hence is essential to a fermentation process in suspension.

Although the underlying mechanism for transformation by E1 is complex one hallmark is a most desirable feature: E1A is a strong inducer of cell proliferation and apoptosis whereas E1B proteins efficiently interfere with apoptosis but cannot release restriction on cell cycle control.

Hence, not a single factor but the continuous presence of E1A and E1B proteins are required to sustain the experimentally induced transformed phenotype.

To our knowledge there are only two microbat cell lines known: TB-1 Lu (ATCC number CCL-88) isolated in 1965 from the lung of Tadarida brasiliensis and Mvi/It (ATCC number CRL-6012) from a skin tumor of Myotis velifer incautus.

No megabat cell line is available and certainly no bat cell line derived by targeted immortalization. This is even more regretable as megabats appear, but not microbats, appear to be main or even single reservoir for Ebola and Henipaviruses.

According to a preferred embodiment of aspect (1) of the invention, the cell line is derived from a primary bat cell. Particularly preferred are fetal or neonatal cells, in particular from neuronal tissue. According to a further preferred embodiment, the cell line is derived from a fruit bat (Megachiroptera), preferably from a Rousettus aegyptiacus. Wild fruit bat were not utilized as donors, but Rousettus aegyptiacus fetuses were received as a gift from a healthy population at the Wilhelma Zoo in Stuttgart, Germany. The Egyptian fruit bat is of a dark brown or reddish brown fur color with a paler shade on the underside. Typical for a megachiropteran, the Egyptian fruit bat has large eyes, a long fox-like muzzle and the face is without prominent features such as nose leaves. Adult bats are up to 15 cm long with a wing span of 40 cm and body weight of 85 to 175 grams. The Egyptian fruit bat is not endangered, breeds readily in captivity and is considered a pest in some regions in the Middle East.

Fetal cells were immortalized by liposomal transfection of expression plasmids for E1 genes. Retroviral transduction was not performed as this procedure is difficult to reconcile with DRA as genetic elements may become mobilized by long terminal repeats.

Transfected cells were cultivated until primary cells died from senescence and culture shock leaving foci of immortalized cells. These were analyzed by immunofluorescence for E1A protein: all cells that could be passaged beyond the life span of primary cells are E1A positive indicating desired targeted (rather than spontaneous) transformation.

In a particularly preferred embodiment of aspect (1) of the invention the cell line is a primary cell of Rousettus aegyptiacus immortalized by an adenoviral E1 gene, preferably is a fetal cell carrying nt 3524 to nt 8361 of SEQ ID NO:1, most preferably is cell line AGE1.R06E as deposited under DSM ACC...

An important property of cells intended as diagnostic tool for infectious diseases or as producer cell for vaccine viruses is the complexity and strength of the innate immune system. A strong innate immune system may render the cell refractory to a number of pathogens and would interfere with attenuation (although species-dependent attenuation still would be available). The innate immune system relies on a number of molecules adapted to recognize molecular signatures of common pathogens. These signatures (or patterns) are chemical compositions usually not found in the healthy organism such as lipopolysaccharides of the bacterial cell wall or double stranded RNA as a byproduct of viral replication. The sensors for these patterns are encoded in a superfamily of genes (together with interleukin 1 receptors) that are called are toll-like receptors (TLRs). Each TLR is a sentinel for a specific pathogen-associated pattern and ultimately communicates detection into the interferon type I pathway. Interferon type I is a paracrine and autocrine factor that mediates death of an infected cell via protein synthesis shutdown, RNA degradation, and other mechanism if a pathogen persists.

Reading the literature it is fair to assume that the innate immune system of bat cells is not unique: TLRs constitute an ancient gene family present already in prototypical vertebrates with their function as sentinels for pathogen associated patterns (Roach et al. 2005 in Proc. Natl. Acad. Sci. U.S.A. 102, 9577-9582). The interferon signaling cascade into an important effector, the 2'-5' oligoadenylate synthetases, is present even in birds and components thereof have been found reptiles and amphibians (Kumar et al. 2000 in Mol. Biol. Evol. 17, 738-750).

Indeed, primary bat cells react with interferon expression to induction with polyinosinic-polycytidylic acid (poly IC) (Omatsu et al. 2006 in Comp. Immun. Microb. & Inf. Dis. 30, 357-374). Poly IC is an artificial double stranded RNA molecule and as such a surrogate pathogen-associated pattern molecule.

Bat cells apparently are not compromised in their innate immune system. It therefore comes as a tremendous surprise that the highly attenuated MVA virus replicates to excellent titers in bat cells. The levels obtained with MVA on Rousettus cells exceed the titers obtained with chicken embryo fibroblasts, the host cell MVA has been adapted to in the course of over 500 passages.

To our knowledge it has never been attempted and described to grow a poxvirus in bat cells, especially not the highly attenuated MVA virus.

MVA is attenuated to such an extent that it cannot replicate in human cells. However, it induces a strong immune response in the human organism and therefor is one of the most promising tools for therapeutic and protective vaccine applications even in immunocompromised patients such as HIV-infected individuals with clinical AIDS or immunosuppressed cancer or transplantation patients.

MVA was generated from vaccinia (cowpox) virus by passaging the virus more than 500 times in primary chicken fibroblasts. During the process a number of mutations and deletions in the viral genome accumulated resulting in severe host restriction. Except for avian cells only BHK-21 (baby hamster kidney) has been shown to be permissive for MVA (Drexler et al. 1998 in J. Gen. Virol. 79, 347-52).

MVA is commercially produced in primary fibroblasts isolated from embryonated chicken eggs. Chicken eggs used for human vaccine production must be certified to be free of a defined set of viral and bacterial contamination (specific pathogen-free or SPF). SPF embryonated eggs are expensive and can constitute up to 40% of the cost of vaccines. It is difficult to continually maintain SPF flocks completely free of pathogens which is evidenced by periodic outbreaks of disease in these flocks. Chicken embryonic fibroblasts are prepared from SPF eggs by mincing embryos to establish and amplify viable cells. Typical for primary animal cells the fibroblasts suffer senescence: the doubling time increases with passaging and eventually all cells die. This process occurs after about 20 passages, much earlier than for rodent or some human cell substrates currently used in vaccine manufacture (such as MRC-5 or WI-38). Fibroblast cultures have to be maintained in the presence of 5-10% fetal calf serum, adding additional risk factors to the manufacturing process. They also require a solid surface for propagation and do not grow in suspension, a preferred state for bioreactor applications. Due to the limited live span a complete set of safety tests has to be applied for each lot of chicken fibroblasts.

A pharmaceutically accepted avian cell line is not yet available although at least one such cell line has been characterized to such an extent that it should be clinically acceptable soon.

BHK-21 easily grow in large fermenters on carriers under serum-free conditions (Pay, T.W. et al., Dev. Biol. Stand 60:171-4 (1985); Gallegos Gallegos, R. M. et al., Arch. Med. Res. 26:59-63 (1995)). The BHK-21 cell line is accepted for production of certain vaccines for livestock animals (Lubiniecki, A.S., Bioprocess Technol. 10:495-513 (1990)). However, the BHK-21 line does not meet the safety requirements for human live vaccines. BHK cells have spontaneously formed, are highly tumorigenic and their history is inadequately reported.

Even more significantly: the MVA titers obtained with BHK-21 were significantly lower compared to the yield obtained with chicken embryo fibroblasts. Because MVA does not replicate in the human organism it must be given at high doses to recipients. Current estimates are 10⁸ infectious units/dose. The titers reported on BHK-21 are in the range of only 10⁶ infectious units/ml.

An additional cell line suitable for clincall production of MVA would be highly desriable. To have an additional line available from a host other than birds would provide unexpected potential to therapeutic procedures and attenuation regimes of MVA.

The ivention is further explained by the following examples, which are, however, not to be construed as a limitation of the invention.

### Examples

### Example 1: Immortalization of primary Rousettus cells with E1 genes

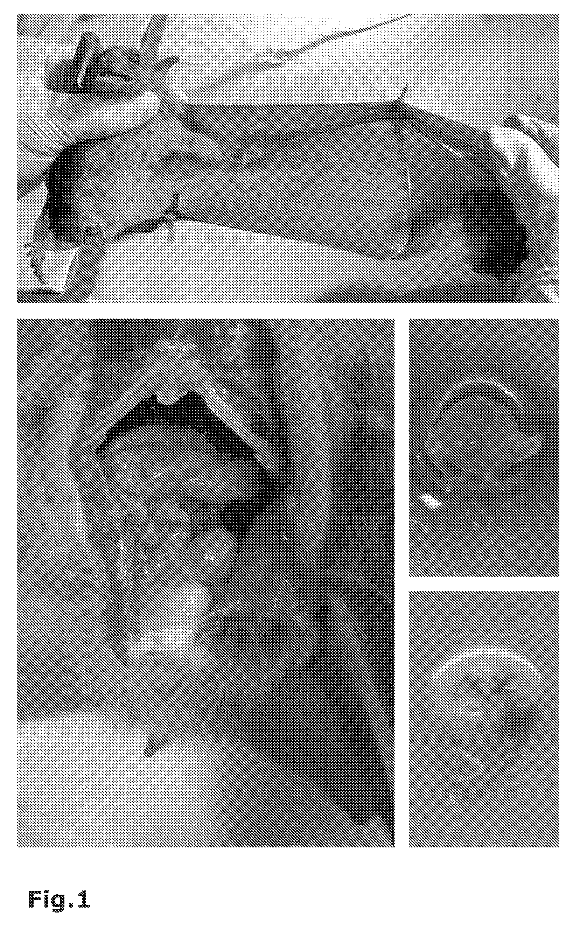

Fetuses from Rousettus aegyptiacus were a kind gift from the Wilhelma Zoo in Stuttgart. Fig. 1 shows steps towards isolation of the fetuses from pregnant bats. The fetuses were separated into head and body and individual cells isolated by brief digestion with TrypLE (Gibco, a trypsin replacement) and tituration into DMEM/F12 medium (Gibco) containing 5% fetal calf serum (Biochrom AG). The cells were seeded into 6-well culture plates and transfected with effectene (Qiagen) within 1 to 1.5 weeks after plating. Cultivation in all of the described experiments was performed at 37°C and 8% CO2 in the gaseous phase.

The plasmid used for transfection and immortalization designated #G56 (Fig. 2 and SEQ ID NO:1) was constructed in accordance with WO/2005/042728 with genomic human adenovirus sertype 5 sequences from a wild-type contamination in a vector preparation produced on HEK 293 cells: primers ACTCGAGCTGACGTGTAGTGTATT and CACACGCAATCACAGGTT (SEQ ID NO:2 and 3, respectively) were used to amplify the E1A region and primers ACTCGAGTCATGGAGGCTTGGGAGT and ACACATTTCAGTACCTCA (SEQ ID NO:4 and 5, respectively) were used to amplify the E1 B region. Amplification was performed with ProofStart polymerase (Qiagen) and integrity confirmed by sequencing.

Expression of E1A is driven by the promoter for human phosphoglycerate kinase, amplfied from genomic DNA of HEK 293 with primers GAGATTAATGGTTGGGGTTGCGCCTT and AACTCGAGAACGAGGGAGCCGACTGCC (SEQ ID NO:6 and 7, respectively). Expression of E1B is driven by the thymidine kinase promoter of herpes simplex virus.

Prior to transfection #G56 was digested with ApaLI, RsrII and ScaI (all from New England Biolabs) restriction enzymes to linearize the plasmid and to remove the bacterial selection markers. Transfection of primary cells was performed with cells growing as monolayers in 6-well plates with 2 µg of linearized plasmid DNA and 16 µl enhancer in Qiagen-provided EC-buffer. After 5 min of incubation, depending on confluency of the cell layer, 16 µl to 20 µl of effectene reagent was added and this suspenson was incubated for further 10 min. Thereafter, the transfection mix was applied to the cell monolayer in a final volume of 1 ml of medium. After 3 hours additional 1.5 ml medium were added, the following day the medium was replaced completely.

Liposomal transfection of primary cells can be difficult. To determine transfection efficiency an expression plasmid for GFP was transfected in parallel (Fig. 2)transfection efficiency was low but adequate. After approx. 2 weeks in culture foci appeared where cells were more homogenous in appearance and smaller in size compared to the large and pleomorphic primary cells (Fig. 2). Because primary cells and transfected cells that did not integrate the DNA together constitute the vast majority in the cultures a considerable hurdle in generation of a new cell line is rescue of the clonal populations. We recovered foci by gentle treatment with TrypLE for a time span that allowed detachment of clonal cells leaving most of the primary cells in the flask. Many primary cells still were transferred but were sequentially lost to senescence over a period of approximately six months of continous culture. Senescence describes a cellular state with decreased proliferation rates until complete stasis or cell death due to telomere erosion of the chromosomes and accumulation of signal proteins important in cell cycle control. Senescent cells usually are large and pleomorphic with striated cytoplasm of low phase contrast.

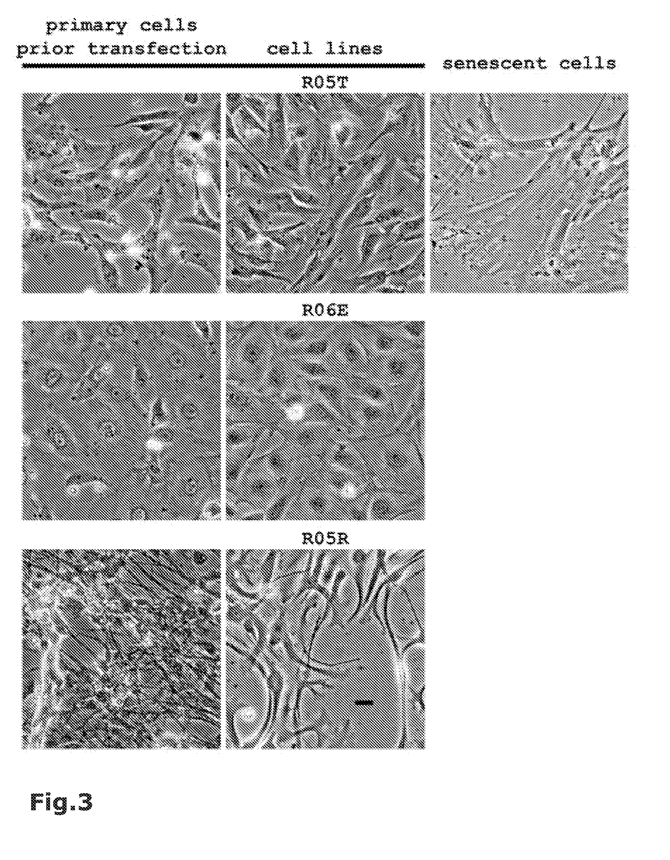

Fig. 3 shows three of the final cell lines after nine months of continous culture in the middle panel. A typical senescent cells is shown in the right panel. Most surprising is the fact that we established three different cell lines of distinct appearance and possibly lineage. The reason for our surprise is based on the fact that E1 immortalization appears to be most efficient in neuronal cells and on an earlier observation suggesting that immortalization of human cells with E1-genes induces a shift in gene expression towards epithelial patterns. We obtained one cell line consistent with epithelial properties and one cell line with fibroblast appearance. Both cell lines highly resemble the source material prior to transfection shown in the left panel of Fig. 3.

Neuronal cells are extremely difficult to cultivate (for example, see Brewer and Cotman 1989 in Brain Research 494, 65-74: they require carefully adjusted hypoxic culture conditions, low culture volumes for self-conditioning of medium and detoxification in absence of supporting glial cells). We did not adapt culture conditions to neuronal cells and to our knowledge it is completely unexpected that neurons continously and without exogenous nerve growth factors or phorbol esters form the long and branched spines as observed in our culture (bottom row of Fig. 3). The primary neuronal cells in the left panel were isolated from the vertebrate of a Rousettus embryo, the immortalized neuronal line was obtained by transfection of cells isolated from the head.

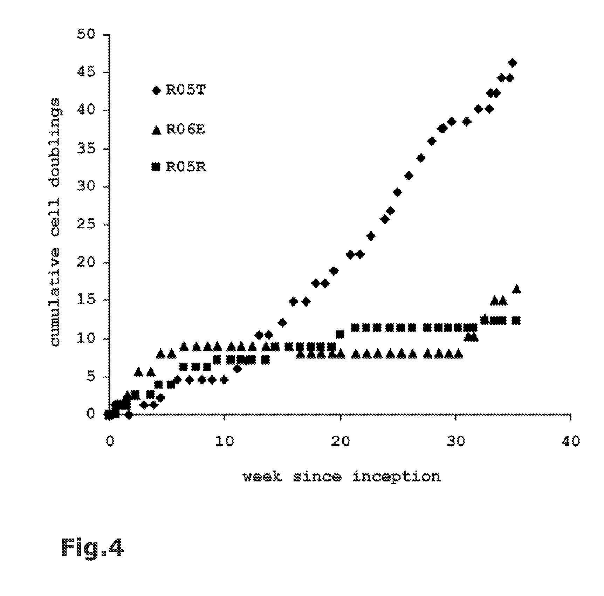

Consistent with culturing properties of true neurons the cell line proliferates very slowly and passaging is possible only once or twice per month. Fig. 4 compares a cell line histories in terms of cumulative cell doublings. Each manipulation event including change of medium has been documented and is depicted here for an intervall of 35 weeks. Clearly visible is the early and strong proliferation of the fibroblast line, stabilization of the epithelial line approx. 30 weeks after isolation and transfection of primary cells, and the slow but successfull cultivation of the neuronal line.

It is a completely unexpected observation of this invention that tissue-specific expression patterns appear not to change on a macroscopic scale in bat cells upon immortalization with E1 genes. This property probably extends to other immortalization strategies.

### Example 2: Immunofluorescence assay for stable transfection

Cultures of immortalized cells were seeded on glass slides and allowed to proliferate for several days before fixation with ice-cold methanol for 10 min. The fixed cells were incubated with antibodies against E1A protein, secondary antibodies, and fluorescent dye specific against the latter according to standard immunofluorescene methods (Becton Dickinson, UK, #554155 antibody against E1A, diluted 1:30; secondary antibody directed against mouse and conjugated to biotin, both from Jackson Immuno Research, USA, diluted 1:80; visualization with Jackson Immuno Research, USA, #016-070-084 streptavidin-Texas Red conjugate, diluted 1:100). AGE1.CR cells (duck retina cells; WO/2005/042728) that stably express the Ad5 E1-region served as positive control, BHK cells as negative control. DAPI (4',6-diamidino-2-phenylindol; Sigma, USA) to 1 µg/ml was added in the final incubation step to stain the nuclei of the cells for orientation purposes.

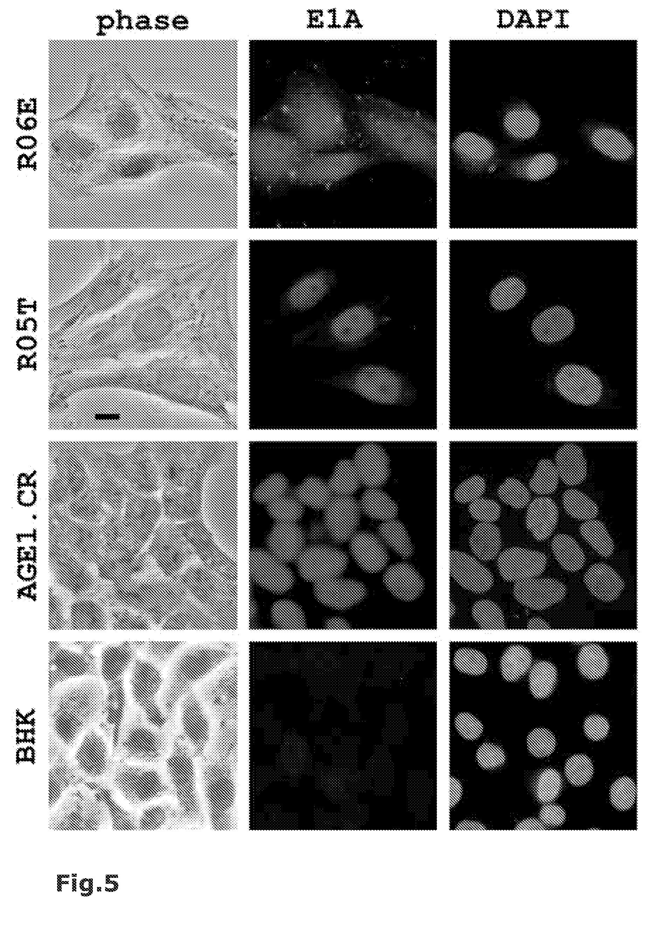

Fig. 5 shows the result obtained with cultures R05T and R06E. A strong signal for E1A was observed in all cells confirming successful immortalization by the transfected plasmids. Furthermore, spontaneous transformation, a formal possibility, was not observed as all cells were E1-positive.

### Example 3: Modified vaccinia virus Ankara (MVA)

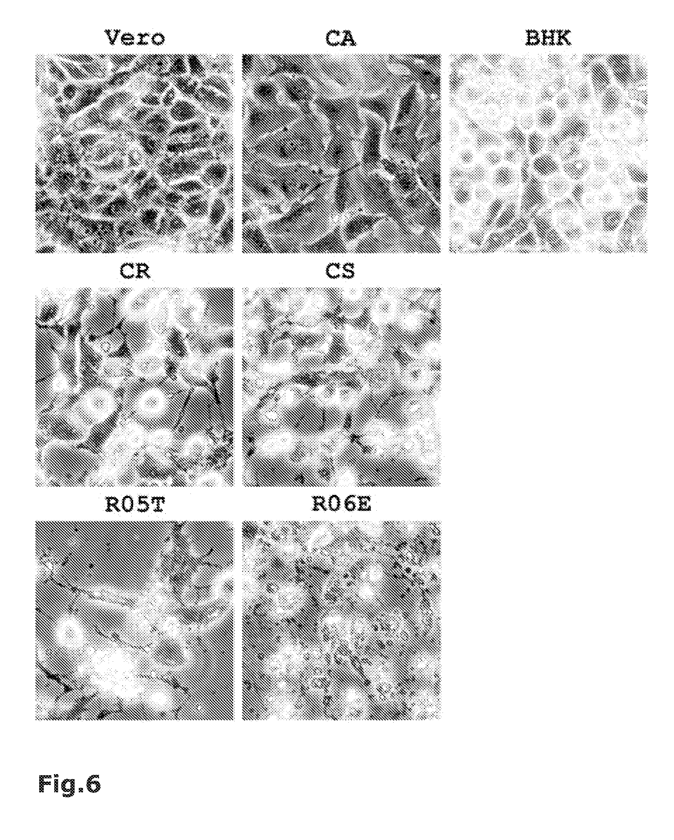

Cells of the Rousettus epithelial and the fibroblast cell lines were seeded into 6-well plates infected with MVA (ATCC #VR-1508) at multiplicity of 0.1 infectious units per cell after 24 hours of culture. As positive controls CR and CS (avian cell lines derived from retina and somites) and as negative controls Vero (African green monkey kidney cells; ATCC CCL-81) and CA (avian amniocyte cell line; patent application WO/2005/042728) were infected in parallel. 48 hours post infection a strong cytopathic effect was evident in the positive controls and suprisingly also in the Rousettus cell lines (Fig. 6). As expected, no spread of virus and no or minimal cytopathic effect was visible in the Vero and CA cell lines that are refractory for MVA.

This result is unexpected as MVA is derived from vaccinia virus Duke strain by adaptation to embryonated chicken eggs (yielding CVA, chorioallantois vaccinia Ankara) followed by more than 500 passages in chicken embryonic fibroblasts (yielding MVA). MVA is attenuated to such an extent that it cannot replicate in mammalian cells tested thus far with BHK as a single exception. The severe host range restriction is programmed into MVA by a number of mutations and deletions that have reduced the size of the genomic DNA from 200 kb in Duke to 192 kb in CVA down to 178 kb in MVA.

Infected cells were resuspended into the culture medium by pipetting and lysed by three cycles of freeze/thawing to also harvest virus from within the cells. The suspension was cleared by centrifugation and titration was performed on Vero cells (Vero cells do not replicate MVA but they are susceptible and can be infected for titration purposes), briefly: Vero cells were seeded in 96 well plates at 2 x 10^4 cells per well and infected with serial 10-fold dilutions of MVA-containing suspension on the following day. Two days thereafter, the cultures were fixed with methanol and incubated with polyclonal vaccinia virus antibodies (Quartett, Germany, #9503-2057, at 1:1000 dilution in PBS containing 1% fetal calf serum) for 1 hour at 37°C. Two wash steps were performed with PBS containing 0.05% Tween 20 (Sigma Corp, USA) and secondary antibody to the vaccinia-specific antibody is added at 1:1000 dilution in PBS containing 1% fetal calf serum. This secondary antibody is coupled to the peroxidase enzyme that catalyzes a color reaction upon incubation with AEC reagent (3-amino-9-ethyl-carbozole; 0.3 mg/ml in 0.1 M acetate buffer pH 5.0 containing 0.015% H₂O₂). Infected foci are identified by light microscopy and plaque forming units are calculated from the maximum dilution of MVA suspension that yields a positive dye reaction.

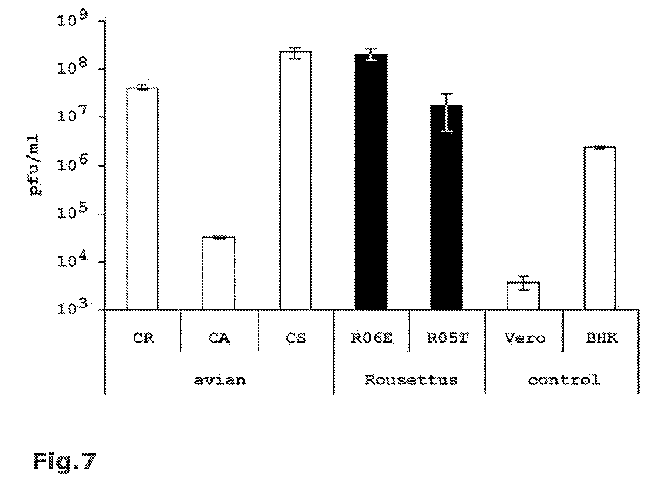

Fig. 7 demonstrates that replication of MVA in Rousettus cells is not an artefact but clearly surpasses the published values in the range of 10⁶ pfu/ml for replication in BHK (Drexler et al. 1998 in J. Gen. Virol. 79, 347-52). Yields for MVA in multiplying in the avian cell line AGE1.CS is supperior to yields in primary chicken embryo fibroblasts, an accepted production system for MVA. R06E releases similar levels of MVA (2.3 x 10⁸ pfu/ml and 2.1 x 10⁸ pfu/ml, respectively). The R05T cell line is less efficient than R06E but with 3.3 x 10⁷ pfu/ml in the expected range for a fully permissive host cell and similar in yields to the permissve avian cell line AGE1.CR. Nonpermissive cell lines CA and Vero provide a base line: yields there correspond to input virus or extremely limited replication in the case of the CA line. The literature value for BHK is confirmed in our experiments performed in parallel to the infection of the novel Rousettus cell lines: BHK produces less virus than R05T and R06E.

For production purposes of vaccine vectors one often calculates burst rates given by the ratio of yield to inocolum with seed virus. Burst size is equivialent to amplification of virus and thus important to estimate cost and required resources for large scale production.

For R06E the burst size is 4133 at a multiplicity of infection with 0.1, an excellent amplification for MVA. High amplification rates are especially important for MVA because this vector cannot replicate in the human vaccinée. This is an important safety feature as even immunocompromised patients can receive MVA as therapeutic vaccine. However, lack of replication necessitates vaccination with high doses of infectious particles -- most estimates are in the range of 10⁸ infectious units/shot. Providing a mammalian cell line generated according to the defined risk approach that allows economical production of MVA for vaccine purposes is highly desirable.

### Example 4: poly IC

In a recent publication on bat microbiology and epidemiology (Omatsu et al. 2006 in Comp. Immun. Microb. & Inf. Dis. 30, 357-374) it is strongly suggested "to use primary cell culture [rather] than using an established cell line to evaluate host response to virus or microbes." because the only available bat cell line TB-1 Lu is deficient in response to dsRNA.

dsRNA is one of several pathogen associated patterns a cell uses to identify exposure to parasites. These molecular patterns are recognized by sensor molecules called Toll-Like Receptors or TLRs. TLRs communicate into the interferon type I pathways. These interferons induce an anti-viral state in non-infected cells and apoptosis in infected cells, thus precluding viral replication.

Thirteen TLRs are known, each receptor with specificity for a class of molecular patterns such as bacterial lipopolysaccharides (TLR-4), glycolipids (TLR-2), flagellin (TLR-5), unmethylated CpG-DNA (TLR-9) and dsRNA (TLR-3). If a cell does not respond to a given pattern the innate immune system is compromized, either because of properties of the immortalized source material as different tissues are differently equipped to respond to pathogens, or because the immortalization events have disabled certain biochemical pathways. This apparently has occured in the TB-1 Lu compromising suitability of this cell line for diagnostic purposes that rely on cytopathic effect and for research where biochemical pathways need to be characterized.

Primary cell cultures are not a desirable alternative to TB-1 Lu: they require continous supply of donor animals which is difficult especially in the case of bats, and often introduce great variations in cell properties with each preparation and as passage number increases.

To determine whether our Rousettus cell lines respond to dsRNA we used a common synthetic analogue of viral dsRNA, poly IC (Sigma), and compared effects of treatment with poly IC to a cell line known to be sensitive to TLR-3 induction (AGE1.CS) and to a cell known to be deficient in interferon type I pathways (Vero). Poly IC was added at a concentration of 400 µg/ml directly to the medium of the cells in the presence of fetal calf serum to challenge the TLR-3 receptor; poly IC can also be transfected to interrogate intra-cellular receptors for dsRNA but this would introduce the molecular pattern downstream of the interferon induction and is a less stringent examination.

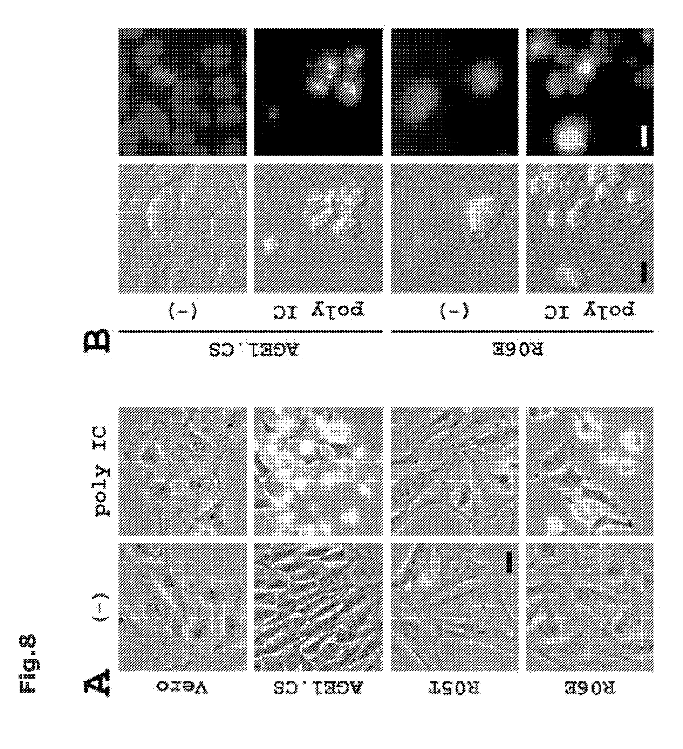

Within 8 hours of treatment increased cell death was observed for the CS and the R06E cell lines (Fig. 8, panel A). To confirm that cell death is due to apoptosis the culture was stained with 5 µg/ml DAPI without prior fixation. Fragmented nuclei were observed only in poly IC treated CS and R06E cell lines (Fig. 8, panel B) demonstrating induction of apoptosis and thus intact TLR-3 signaling into the interferon pathways (for example, see DeWitte-Orr et al. 2005 in Fisch & Shellfish Immunology 18, 279-295, and Tanaka et al. 1998 in Genes to Cell 3, 29-37 for demonstration that interferon is required for induction of apoptosis by dsRNA). Failure of R05T and Vero to respond similarily to dsRNA indicates that poly IC per se is not toxic to the cells and that we can both provide a highly desirable novel cell line responsive to at least one pathogen associated molecular pattern (R06E) and another novel cell line from Rousettus (R05T) as direct comparision to TB-1 Lu from a microbat.

In conclusion, we generated at least three different cell lines with distinct properties from tissues of Rousettus aegyptiacus fetuses. The cell lines are unusual in that they appear to have retained a number of primary features that even extend to a neuronal line. The lines are also unusual in that they are fully permissive for MVA, a highly attenuated virus that usually does not replicate in mammalian cells. Furthermore, at least one of the cell lines is unusual in that it responds with interferon induction to challenge with a pathogen associated molecular pattern.

Bats appear to be unique among mammals because they rarely exhibit clinical symptoms after infection with a variety of extremely virulent pathogens such as Ebola virus, SARS-CoV and lyssavirus. We add a paradox to this observation: bat cells appear not to be especially resistant to pathogens. Rather, they replicate highly attenuated MVA to surprisingly high levels that rival yields obtained on cells of the avian host MVA has been adapted to.

### Sequence Listing (Free Text)

| | |
|---|---|
| SEQ ID NO:1 | transfection vector #G56 |
| SEQ ID NOs:2-7 | primers |

## Claims

1. A cell line derived from a bat (Chiroptera) cell immortalized by a defined mechanism.

2. The cell line of claim 1 which is derived from
(i) a primary bat cell, preferably from a fetal or neonatal cell, preferably from neuronal tissue; and/or
(ii) a fruit bat (Megachiroptera), preferably from a Rousettus aegyptiacus.

3. The cell line according to claim 1 or 2, which additionally carries at least one non-native functional sequence including, but not limited to, transgenes such as genes complementing deficient viruses (such as a EBNA1 transactivator or a viral structural protein), promoters (such as PGK-, EF1.alpha-, CMV- and tk-promoters), enhancers (such as RSV-LTR), selection markers (such as neomycin-resistance and puromycin-resistance), reporter genes (such as GFP or lacZ), and/or therapeutic genes (such as antibody genes).

4. The cell line according to anyone of claims 1 to 3 that is missing genes (e.g. via knock-out), and/or that is blocked or is reduced in gene expression (e.g. via gene silencing).

5. The cell line according to anyone of claims 1 to 4, which
(i) if contacted with a microbial agent, preferrably a virus or viral vector, replicates the microbial agent; and/or
(ii) proliferates in medium free of animal-derived components.

6. The cell line according to any one of claims 1 to 4,
which is a primary cell of Rousettus aegyptiacus immortalized by an adenoviral E1 gene, preferably is a fetal cell carrying nt 3524 to nt 8361 of SEQ ID NO:1, most preferably is cell line AGE1.R06E deposited under DSM ACC...

7. A method for preparing the cell line of claims 1 to 6, which comprises immortalizing a starting bat cell.

8. A method for producing a microbial agent on a cell line according to anyone of claims 1 to 6, which comprises contacting said cell line with
(i) the microbial agent, including infection with the microbial agent, cultivating the infected cells, harvesting the cells or the culture supernatant to retrieve the microbial agent; or
(ii) with a nucleic acid sequence encoding said microbial agent, including transfection or transduction with an expression plasmid or in vitro transcribed RNA, cultivating the transfected cells, harvesting the cells or the culture supernatant to retrieve the microbial agent.

9. A method for diagnosis, identification, retrieval or rescue of a microbial agent which includes exposing the cells from a cell line according to anyone of claims 1 to 6 to medium or biological samples suspected to contain a certain microbial agent.

10. The method of claim 8 or 9, wherein the microbial agent is a virus or viral vector, preferably a modified vaccinia Ankara.

11. Use of a cell line according to anyone of claims 1 to 6 for production, diagnosis, identification, retrieval or rescue of a microbial agent, or for identification of at least one factor important for replication of or resistance against a microbial agent, and application or exogenous expression of this factor or these factors in a cell that is not isolated from a chiropteran.


SEQUENCE LISTING

<110>  ProBioGen AG

<120>  Cell Line from Rousettus as Host Cell for Pathogen Amplification

<130>  080479ep

<160>  7

<170>  PatentIn version 3.3

<210>  1
<211>  8681
<212>  DNA
<213>  Artificial

<220>
<223>  transfection vector # G56

<400>  1
cttccgcttc ctcgctcact gactcgctgc gctcggtcgt tcggctgcgg cgagcggtat      60

cagctcactc aaaggcggta atacggttat ccacagaatc aggggataac gcaggaaaga     120

acatgtgagc aaaaggccag caaaaggcca ggaaccgtaa aaaggccgcg ttgctggcgt     180

ttttccatag gctccgcccc cctgacgagc atcacaaaaa tcgacgctca agtcagaggt     240

ggcgaaaccc gacaggacta taaagatacc aggcgtttcc ccctggaagc tccctcgtgc     300

gctctcctgt tccgaccctg ccgcttaccg gatacctgtc cgcctttctc ccttcgggaa     360

gcgtggcgct ttctcatagc tcacgctgta ggtatctcag ttcggtgtag gtcgttcgct     420

ccaagctggg ctgtgtgcac gaaccccccg ttcagcccga ccgctgcgcc ttatccggta     480

actatcgtct tgagtccaac ccggtaagac acgacttatc gccactggca gcagccactg     540

gtaacaggat tagcagagcg aggtatgtag gcggtgctac agagttcttg aagtggtggc     600

ctaactacgg ctacactaga aggacagtat ttggtatctg cgctctgctg aagccagtta     660

ccttcggaaa aagagttggt agctcttgat ccggcaaaca aaccaccgct ggtagcggtg     720

gtttttttgt ttgcaagcag cagattacgc gcagaaaaaa aggatctcaa gaagatcctt     780

tgatcttttc tacggggtct gacgctcagt ggaacgaaaa ctcacgttaa gggattttgg     840

tcatgagatt atcaaaaagg atcttcacct agatcctttt aaattaaaaa tgaagtttta     900

aatcaatcta aagtatatat gagtaaactt ggtctgacag ttaccaatgc ttaatcagtg     960

aggcacctat ctcagcgatc tgtctatttc gttcatccat agttgcctga ctccccgtcg    1020

tgtagataac tacgatacgg gagggcttac catctggccc cagtgctgca atgataccgc    1080

gagacccacg ctcaccggct ccagatttat cagcaataaa ccagccagcc ggaagggccg    1140

agcgcagaag tggtcctgca actttatccg cctccatcca gtctattaat tgttgccggg    1200

aagctagagt aagtagttcg ccagttaata gtttgcgcaa cgttgttgcc attgctacag    1260

gcatcgtggt gtcacgctcg tcgtttggta tggcttcatt cagctccggt tcccaacgat 1320
caaggcgagt tacatgatcc cccatgttgt gcaaaaaagc ggttagctcc ttcggtcctc 1380
cgatcgttgt cagaagtaag ttggccgcag tgttatcact catggttatg gcagcactgc 1440
ataattctct tactgtcatg ccatccgtaa gatgcttttc tgtgactggt gagtactcaa 1500
ccaagtcatt ctgagaatag tgtatgcggc gaccgagttg ctcttgcccg gcgtcaatac 1560
gggataatac cgcgccacat agcagaactt taaaagtgct catcattgga aaacgttctt 1620
cggggcgaaa actctcaagg atcttaccgc tgttgagatc cagttcgatg taacccactc 1680
gtgcacccaa ctgatcttca gcatctttta ctttcaccag cgtttctggg tgagcaaaaa 1740
caggaaggca aaatgccgca aaaaagggaa taagggcgac acggaaatgt tgaatactca 1800
tactcttcct ttttcaatat tattgaagca tttatcaggg ttattgtctc atgagcggat 1860
acatatttga atgtatttag aaaaataaac aaataggggt tccgcgcaca tttccccgaa 1920
aagtgccacc tgtatgcggt gtgaaatacc gcacagatgc gtaaggagaa aataccgcat 1980
caggaaattg taagcgttaa taattcagaa gaactcgtca agaaggcgat agaaggcgat 2040
gcgctgcgaa tcgggagcgg cgataccgta aagcacgagg aagcggtcag cccattcgcc 2100
gccaagctct tcagcaatat cacgggtagc caacgctatg tcctgatagc ggtccgccac 2160
acccagccgg ccacagtcga tgaatccaga aaagcggcca ttttccacca tgatattcgg 2220
caagcaggca tcgccatggg tcacgacgag atcctcgccg tcgggcatgc tcgccttgag 2280
cctggcgaac agttcggctg gcgcgagccc ctgatgctct tcgtccagat catcctgatc 2340
gacaagaccg gcttccatcc gagtacgtgc tcgctcgatg cgatgtttcg cttggtggtc 2400
gaatgggcag gtagccggat caagcgtatg cagccgccgc attgcatcag ccatgatgga 2460
tactttctcg gcaggagcaa ggtgagatga caggagatcc tgccccggca cttcgcccaa 2520
tagcagccag tcccttcccg cttcagtgac aacgtcgagc acagctgcgc aaggaacgcc 2580
cgtcgtggcc agccacgata gccgcgctgc ctcgtcttgc agttcattca gggcaccgga 2640
caggtcggtc ttgacaaaaa gaaccgggcg cccctgcgct gacagccgga acacggcggc 2700
atcagagcag ccgattgtct gttgtgccca gtcatagccg aatagcctct ccacccaagc 2760
ggccggagaa cctgcgtgca atccatcttg ttcaatcatg cgaaacgatc ctcatcctgt 2820
ctcttgatca gagcttgatc ccctgcgcca tcagatcctt ggcggcgaga aagccatcca 2880
gtttactttg cagggcttcc caaccttacc agagggcgcc ccagctggca attccggttc 2940
gcttgctgtc cataaaaccg cccagtctag ctatcgccat gtaagcccac tgcaagctac 3000
ctgctttctc tttgcgcttg cgttttccct tgtccagata gcccagtagc tgacattcat 3060
ccggggtcag caccgtttct gcggactggc tttctacgtg aaaaggatct aggtgaagat 3120
cctttttgat aatctcatgc ctgacattta tattccccag aacatcaggt taatggcgtt 3180

tttgatgtca ttttcgcggt ggctgagatc agccacttct tccccgataa cggagaccgg 3240

cacactggcc atatcggtgg tcatcatgcg ccagctttca tccccgatat gcaccaccgg 3300

gtaaagttca cgggagactt tatctgacag cagacgtgca ctggccaggg ggatcaccat 3360

ccgtcgcccc ggcgtgtcaa taatatcact ctgtacatcc acaaacagac gataacggct 3420

ctctctttta taggtgtaaa ccttaaactg ccgtaccatg ttctttcctg cgttatcccc 3480

tgattctgtg gataaccgta ttaccgccat gcattagtta ttaatggttg gggttgcgcc 3540

ttttccaagg cagccctggg tttgcgcagg gacgcggctg ctctgggcgt ggttccggga 3600

aacgcagcgg cgccgaccct gggtctcgca cattcttcac gtccgttcgc agcgtcaccc 3660

ggatcttcgc cgctaccctt gtgggccccc cggcgacgct tcctcgtccg cccctaagtc 3720

gggaaggttc cttgcggttc gcggcgtgcc ggacgtgaca aacggaagcc gcacgtctca 3780

ctagtaccct cgcagacgga cagcgccagg gagcaatggc agcgcgccga ccgcgatggg 3840

ctgtggccaa tagcggctgc tcagcagggc gcgccgagag cagcggccgg gaaggggcgg 3900

tgcgggaggc ggggtgtggg gcggtagtgt gggccctgtt cctgcccgcg cggtgttccg 3960

cattctgcaa gcctccggag cgcacgtcgg cagtcggctc cctcgttctc gagttatagt 4020

cagctgacgt gtagtgtatt tatacccggt gagttcctca agaggccact cttgagtgcc 4080

agcgagtaga gttttctcct ccgagccgct ccgacaccgg gactgaaaat gagacatatt 4140

atctgccacg gaggtgttat taccgaagaa atggccgcca gtcttttgga ccagctgatc 4200

gaagaggtac tggctgataa tcttccacct cctagccatt ttgaaccacc taccettcac 4260

gaactgtatg atttagacgt gacggccccc gaagatccca acgaggaggc ggtttcgcag 4320

atttttcccg actctgtaat gttggcggtg caggaaggga ttgacttact cacttttccg 4380

ccggcgcccg gttctccgga gccgcctcac ctttcccggc agcccgagca gccggagcag 4440

agagccttgg gtccggtttc tatgccaaac cttgtaccgg aggtgatcga tcttacctgc 4500

cacgaggctg gctttccacc cagtgacgac gaggatgaag agggtgagga gtttgtgtta 4560

gattatgtgg agcaccccgg gcacggttgc aggtcttgtc attatcaccg gaggaatacg 4620

gggggaccag atattatgtg ttcgctttgc tatatgagga cctgtggcat gtttgtctac 4680

agtaagtgaa aattatgggc agtgggtgat agagtggtgg gtttggtgtg gtaatttttt 4740

ttttaatttt tacagttttg tggtttaaag aattttgtat tgtgattttt ttaaaaggtc 4800

ctgtgtctga acctgagcct gagcccgagc cagaaccgga gcctgcaaga cctacccgcc 4860

gtcctaaaat ggcgcctgct atcctgagac gcccgacatc acctgtgtct agagaatgca 4920

atagtagtac ggatagctgt gactccggtc cttctaacac acctcctgag atacacccgg 4980

tggtcccgct gtgccccatt aaaccagttg ccgtgagagt tggtgggcgt cgccaggctg 5040

tggaatgtat cgaggacttg cttaacgagc ctgggcaacc tttggacttg agctgtaaac 5100

gccccaggcc ataaggtgta aacctgtgat tgcgtgtgtg gttaacgcct ttgtttgctg 5160

aatgagttga tgtaagttta ataaagggtg agataatgtt taacttgcat ggcgtgttaa 5220

atggggcggg gcttaaaggg tatataatgc gccgtgggct aatcttggtt acgaattcgt 5280

ttctagtgga tccaaatgag tcttcggacc tcgcgggggc cgcttaagcg gtggttaggg 5340

tttgtctgac gcggggggag ggggaaggaa cgaaacactc tcattcggag gcggctcggg 5400

gtttggtctt ggtggccacg ggcacgcaga agagcgccgc gatcctctta agcacccccc 5460

cgccctccgt ggaggtgggg gtttggtcgg cgggtggtaa ctggcgggcc gctgactcgg 5520

gcgggtcgcg cgccccagag tgtgaccttt tcggtctgct cgcagacccc cgggcggcgc 5580

cgccgcggcg gcgacgggct cgctgggtcc taggctcaat ggggaccgta tacgtggaca 5640

ggctctggag catccgcacg actgcggtga tattaccgga gaccttctgc gggacgagcc 5700

gggtcacgcg gctgacgcgg agcgtccgtt gggcgacaaa caccaggacg gggcacaggt 5760

acactatctt gtcacccgga ggcgcgaggg actgcaggag cttcagggag tggcgcagct 5820

gcttcatccc cgtggcccgt tgctcgcgtt tgctggcggt gtccccggaa gaaatatatt 5880

tgcatgtctt tagttctatg atgacacaaa ccccgcccag cgtcttgtca ttggcgaatt 5940

cgaacacgca gatgcagtcg ggcggcgcgg tcccaggtcc acttcgcata ttaaggtga 6000

cgcgtgtggc ctcgaatacc gagcgaccct gcagcgaccc gcttaacagc gtcaacagcg 6060

tgccgcagat cgctaccgga ctcagatccc ctacctgcgc ctctcttccc cagacctgcg 6120

cgctactgcg gctcgggcgg tcgctcgcct ggctctgctc catttgactg tctgtgtgca 6180

gtcgcagaac ttcgaagagg gttttgcgct ccatccgtgg cgtttcgctt ttgttcggtt 6240

ttgttgttta tttcattttt tttttccgga gagaggcgag gcggtggtcc acacccgccc 6300

gaggaggaag gatctcgagc atggaggctt gggagtgttt ggaagatttt tctgctgtgc 6360

gtaacttgct ggaacagagc tctaacagta cctcttggtt ttggaggttt ctgtggggct 6420

catcccaggc aaagttagtc tgcagaatta aggaggatta caagtgggaa tttgaagagc 6480

ttttgaaatc ctgtggtgag ctgtttgatt ctttgaatct gggtcaccag gcgcttttcc 6540

aagagaaggt catcaagact ttggattttt ccacaccggg gcgcgctgcg gctgctgttg 6600

cttttttgag ttttataaag gataaatgga gcgaagaaac ccatctgagc ggggggtacc 6660

tgctggattt tctggccatg catctgtgga gagcggttgt gagacacaag aatcgcctgc 6720

tactgttgtc ttccgtccgc ccggcgataa taccgacgga ggagcagcag cagcagcagg 6780

aggaagccag gcggcggcgg caggagcaga gcccatggaa cccgagagcc ggcctggacc 6840

ctcgggaatg aatgttgtac aggtggctga actgtatcca gaactgagac gcattttgac 6900

aattacagag gatgggcagg ggctaaaggg ggtaaagagg gagcgggggg cttgtgaggc 6960

tacagaggag gctaggaatc tagcttttag cttaatgacc agacaccgtc ctgagtgtat 7020

tacttttcaa cagatcaagg ataattgcgc taatgagctt gatctgctgg cgcagaagta 7080

ttccatagag cagctgacca cttactggct gcagccaggg gatgattttg aggaggctat 7140

tagggtatat gcaaaggtgg cacttaggcc agattgcaag tacaagatca gcaaacttgt 7200

aaatatcagg aattgttgct acatttctgg gaacggggcc gaggtggaga tagatacgga 7260

ggatagggtg gcctttagat gtagcatgat aaatatgtgg ccgggggtgc ttggcatgga 7320

cggggtggtt attatgaatg taaggtttac tggccccaat tttagcggta cggttttcct 7380

ggccaatacc aaccttatcc tacacggtgt aagcttctat gggtttaaca atacctgtgt 7440

ggaagcctgg accgatgtaa gggttcgggg ctgtgccttt tactgctgct ggaagggggt 7500

ggtgtgtcgc cccaaaagca gggcttcaat taagaaatgc ctctttgaaa ggtgtacctt 7560

gggtatcctg tctgagggta actccagggt gcgccacaat gtggcctccg actgtggttg 7620

cttcatgcta gtgaaaagcg tggctgtgat taagcataac atggtatgtg gcaactgcga 7680

ggacagggcc tctcagatgc tgacctgctc ggacggcaac tgtcacctgc tgaagaccat 7740

tcacgtagcc agccactctc gcaaggcctg gccagtgttt gagcataaca tactgacccg 7800

ctgttccttg catttgggta acaggagggg ggtgttccta ccttaccaat gcaatttgag 7860

tcacactaag atattgcttg agcccgagag catgtccaag gtgaacctga acggggtgtt 7920

tgacatgacc atgaagatct ggaaggtgct gaggtacgat gagacccgca ccaggtgcag 7980

accctgcgag tgtggcggta aacatattag gaaccagcct gtgatgctgg atgtgaccga 8040

ggagctgagg cccgatcact tggtgctggc ctgcacccgc gctgagtttg gctctagcga 8100

tgaagataca gattgaggat aacacggaag gagacaatac cggaaggaac ccgcgctatg 8160

acggcaataa aaagacagaa taaaacgcac ggtgttgggt cgtttgttca taaacgcggg 8220

gttcggtccc agggctggca ctctgtcgat accccaccga gaccccattg gggccaatac 8280

gcccgcgttt cttccttttc cccaccccac cccccaagtt cgggtgaagg cccagggctc 8340

gcagccaacg tcggggcggc agcttaagat taagggcgaa ttcgtttaaa cctgcaggac 8400

tagaaacctg caggactagt ccctttagtg agggttaatt ctgagcttgg cgtaatcatg 8460

gtcatagctg tttcctgtgt gaaattgtta tccgctcaca attccacaca acatacgagc 8520

cggaagcata aagtgtaaag cctggggtgc ctaatgagtg agctaactca cattaattgc 8580

gttgcgctca ctgcccgctt tccagtcggg aaacctgtcg tgccagctgc attaatgaat 8640

cggccaacgc gcggggagag gcggtttgcg tattgggcgc t 8681

<210> 2
<211> 24
<212> DNA

<213>  Artificial

<220>
<223>  primer

<400>  2
actcgagctg acgtgtagtg tatt                                              24


<210>  3
<211>  18
<212>  DNA
<213>  Artificial

<220>
<223>  primer

<400>  3
cacacgcaat cacaggtt                                                     18


<210>  4
<211>  25
<212>  DNA
<213>  Artificial

<220>
<223>  primer

<400>  4
actcgagtca tggaggcttg ggagt                                             25


<210>  5
<211>  18
<212>  DNA
<213>  Artificial

<220>
<223>  primer

<400>  5
acacatttca gtacctca                                                     18


<210>  6
<211>  26
<212>  DNA
<213>  Artificial

<220>
<223>  primer

<400>  6
   gagattaatg gttggggttg cgcctt                                             26


<210>  7
<211>  27
<212>  DNA
<213>  Artificial

<220>

<223>  primer

<400>  7
aactcgagaa cgagggagcc gactgcc                                          27